# EUROPEAN PATENT SPECIFICATION

(11) **EP 0 771 201 B1**
(45) Date of publication and mention of the grant of the patent: **18.04.2001**
(21) Application number: 95930875.0
(22) Date of filing: 21.08.1995
(51) Int. Cl.: A61K 31/4535, A61K 31/4025, A61K 31/381

(54) **USE OF 2-PHENYL-3-AROYLBENZOTHIOPHENES FOR THE PREPARATION OF A MEDICAMENT FOR INHIBITING PRIMARY ENDOMETRIAL HYPERPLASIA**
VERWENDUNG VON 2-PHENYL-3-AROYLBENZOTHIOPHEN DERIVATEN ZUR HERSTELLUNG EINES MEDIKAMENTS ZUR HEMMUNG DER PRIMÄREN ENDOMETRIUMHYPERPLASIE
UTILISATION DES 2-PHENYLE-3-AROYLBENZOTHIOPHENES POUR LA PREPARATION D'UN MEDICAMENT POUR L'EMPECHEMENT DE L'HYPERPLASIE ENDOMETRIALE PRIMAIRE

(30) Priority: 22.08.1994 US 294121
(43) Date of publication of application: 07.05.1997
(73) Proprietor: ELI LILLY AND COMPANY, Indianapolis, Indiana 46285 (US)
(72) Inventor: FUCHS-YOUNG, Robin Sharon Lee, Trafalgar, IN 46181 (US)
(74) Representative: Hudson, Christopher Mark
(86) International application number: US9510619
(87) International publication number: WO9605832

(56) References cited:
- EP-A- 0 652 004
- EP-A- 0 652 005
- WO-A-93/10113
- BREAST CANCER RES. TREAT., vol. 2, no. 3, 1982, NETHERLANDS, page 279 XP002034737 JONES, D., ET AL.: "LY156758:A UNIQUE ANTIESTROGEN DISPLAYING HIGH AFFINITY FOR ESTROGEN RECEPTORS, NEGLIGIBLE ESTROGENIC ACTIVITY AND NEAR-TOTAL ESTROGEN ANTAGONISM IN VIVO."
- ANNALS OF THE NEW YORK ACADEMY OF SCIENCES, vol. 761, 12 June 1995, USA, pages 355-360, XP000654717 FUCHS-YOUNG, R., ET AL.: "RALOXIFENE IS A TISSUE-SELECTIVE AGONIST/ANTAGONIST THAT FUNCTIONS THROUGH THE ESTROGEN RECEPTOR"
- DRUG DEVELOPMENT RESEARCH , vol. 36, no. 1, 1995, USA, pages 43-45, XP000654665 SWISHER K.D., ET AL.: "EFFECT OF THE SELECTIVE ESTROGEN RECEPTOR MODULATOR RALOXIFENE ON EXPLANTED UTERINE GROWTH IN RATS"
- CANCER RES., vol. 50, no. 11, June 1990, USA, pages 3189-3192, XP000654576 GOTTARDIS M.,M., ET AL.: "EFFECT OF STEROIDAL AND NONSTEROIDAL ANTIESTROGENS ON THE GROWTH OF A TAMOXIFENE-STIMULATED HUMAN ENDOMETRIAL CARCINOMA (ENCA101) IN ATHYMIC MICE"
- BREAST CANCER RES. TREAT., vol. 36, no. 3, 1995, NETHERLANDS, pages 267-285, XP000654844 V.CRAIG JORDAN: "STUDIES ON THE ESTROGEN RECPTOR IN BREAST CANCER-20 YEARS AS A TARGET FOR THE TREATMENT AND PREVENTION OF CANCER"

## Description

The uterine lining (endometrium) is composed of tissue, blood vessels, and glands that grow when stimulated by the hormone estrogen. In women with normal menstrual cycles, hormonal fluctuations trigger the growth and shedding of the endometrium each month. If conception occurs, the endometrium nourishes the developing embryo.

With continuously elevated estrogen levels, the endometrium remains in its growth phase at all time, leading to an overabundance of endometrial tissue or endometrial hyperplasia. Overgrowth of the endometrium is often a benign condition, but it can also be a precursor of endometrial cancer. Because of this risk, doctors urge women avoid long-term estrogen therapy, which can cause endometrial overgrowth, and to seek prompt treatment for conditions that cause excessive estrogen production.

Continuous estrogen stimulation of the endometrium without the balancing effects of the hormone progesterone leads to endometrial overgrowth and potentially endometrial cancer. This problem can occur if one begins estrogen replacement therapy after menopause without using progesterone as well; if one is markedly overweight, since excess body fat leads to increased estrogen production; or if one has a condition such as polycystic ovary disease that prevents ones body from maintaining the proper hormonal balance.

Most cases of endometrial carcinoma are associated with a precursor lesion termed "endometrial hyperplasia." The classification of endometrial hyperplasia is based on the presence or absence of cytologic atypia, the presence of dysplasia, and the degree of complexity of the architectural pattern. Cytologic atypia is the most predictive criterion for the likelihood of progression to carcinoma.

In simple or cystic hyperplasia with cytologic atypia present there is about an 8% chance of progression to cancer. With complex or adenomatous hyperplasia with cytologic atypia present, there is 29% chance. When no cytologic atypia is present, the progression rate is 1% for simple and 3% for complex hyperplasia.

Gynecologists diagnose the disorder by examining endometrial tissue taken either as a biopsy or in a procedure called a D&C (dilation and curettage), which involves dilating the cervix and using a sharp, curved instrument to scrape out the entire endometrium. Neither of these procedures is performed routinely. Instead, doctors recommend them for people who have symptoms of endometrial overgrowth or who are at risk of developing it, including post-menopausal women on estrogen therapy and women who are obese or who have fertility problems.

Doctors base their treatment decisions or several factors. First, they examine the cells obtained in the biopsy or D&C. If the cells are normal but simply over abundant, future development of cancer is less likely than if the cells are atypical, displaying enlarged nuclei and other unusual features. In some cases, a D&C will show that cancer has already developed.

The woman's age and desire to have children are also factors to consider. Some doctors advise post-menopausal women with endometrial overgrowth to undergo hysterectomies (surgery to remove the uterus) even if the endometrial cells are not atypical. Others use more conservative treatments, including a D&C (which can be a therapeutic as well as a diagnostic tool) or hormonal therapy. Doctors rarely recommend hysterectomies for younger women unless cancer is actually present or the endometrial cells are extremely atypical.

Women whose endometrial overgrowth is a result of estrogen therapy should add progesterone to their hormone regimens for the last 12 days of each month. After 6 months of this treatment, the doctor will take another endometrial sample. If hyperplasia is still present, the woman can either stop the estrogen or take both estrogen and progesterone throughout the month. If the overgrowth persists, a hysterectomy may be necessary.

The problems with the current therapies, however, indicate a need for a novel method of inhibiting primary endometrial hyperplasia, and its progression to endometrial cancer.

This invention provides the use of a compound of formula I 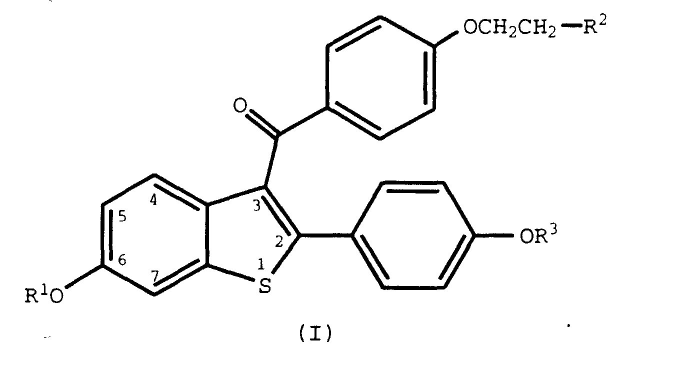 wherein R¹ and R³ are independently hydrogen, -CH₃, 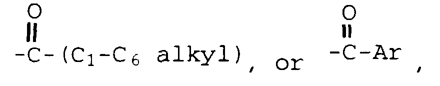 wherein Ar is optionally substituted phenyl;
R² is selected from the group consisting of pyrrolidino, hexamethyleneimino, and piperidino; and pharmaceutically acceptable salts and solvates thereof for the manufacture of a medicament for inhibiting primary endometrial hyperplasia.

Breast Cancer Res. Treat., vol.2, no.3, 1982, NETHERLANDS, page 279; Jones, D., et al.: "LY 156785 : A unique antiestrogen displaying high affinity for estrogen receptors, negligible estrogenic activity and near-total estrogen antagonism in-vivo" (see abstract no. 12; lines 19-21), discloses that raloxifene (=LY 156785) regressed the estrogen-hypertrophied uterus in a manner similar to withdrawal of estradiol treatment.

The current invention concerns the discovery that a select group of 2-phenyl-3-aroylbenzothiophenes (benzothiophenes), those of formula I, are useful for inhibiting primary endometrial hyperplasia.

The therapeutic and prophylactic treatments provided by this invention are practiced by administering to a human in need thereof a dose of a compound of formula I or a pharmaceutically acceptable salt or. solvate thereof, that is effective to inhibit primary endometrial hyperplasia or its symptoms.

The term "inhibit" includes its generally accepted meaning which includes prohibiting, preventing, restraining, and slowing, stopping or reversing progression, severity or a resultant symptom. As such, the present use includes both medical therapeutic and/or prophylactic administration, as appropriate.

Raloxifene is a preferred compound of this invention and it is the hydrochloride salt of a compound of formula 1 wherein R¹ and R³ are hydrogen and R² is 1-piperidinyl.

Generally, at least one compound of formula I is formulated with common excipients, diluents or carriers, and compressed into tablets, or formulated as elixirs or solutions for convenient oral administration, or administered by the intramuscular or intravenous routes. The compounds can be administered transdermally, and may be formulated as sustained release dosage forms and the like.

The compounds used in the methods of the current invention can be made according to established procedures, such as those detailed in U.S. Patent Nos. 4,133,814, 4,418,068, and 4,380,635. In general, the process starts with a benzo[b]thiophene having a 6-hydroxyl group and a 2-(4-hydroxyphenyl) group. The starting compound is protected, acylated, and deprotected to form the formula I compounds. Examples of the preparation of such compounds are provided in the U.S. patents discussed above. The term "optionally substituted phenyl" includes phenyl and phenyl substituted once or twice with C₁-C₆ alkyl, C₁-C₄ alkoxy, hydroxy, nitro, chloro, fluoro, or tri(chloro or fluoro)methyl.

The compounds used in the methods of this invention form pharmaceutically acceptable acid and base addition salts with a wide variety of organic and inorganic acids and bases and include the physiologically acceptable salts which are often used in pharmaceutical chemistry. Such salts are also part of this invention. Typical inorganic acids used to form such salts include hydrochloric, hydrobromic, hydroiodic, nitric, sulfuric, phosphoric, hypophosphoric and the like. Salts derived from organic acids, such as aliphatic mono and dicarboxylic acids, phenyl substituted alkanoic acids, hydroxyalkanoic and hydroxyalkandioic acids, aromatic acids, aliphatic and aromatic sulfonic acids, may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, β-hydroxybutyrate, butyne-1,4-dioate, hexyne-1,4-dioate, caprate, caprylate, chloride, cinnamate, citrate, formate, fumarate, glycollate, heptanoate, hippurate, lactate, malate, maleate, hydroxymaleate, malonate, mandelate, mesylate, nicotinate, isonicotinate, nitrate, oxalate, phthalate, teraphthalate, phosphate, monohydrogenphosphate, dihydrogenphosphate, metaphosphate, pyrophosphate, propiolate, propionate, phenylpropionate, salicylate, sebacate, succinate, suberate, sulfate, bisulfate, pyrosulfate, sulfite, bisulfite, sulfonate, benzene-sulfonate, p-bromophenylsulfonate, chlorobenzenesulfonate, ethanesulfonate, 2-hydroxyethanesulfonate, methanesulfonate, naphthalene-1-sulfonate, naphthalene-2-sulfonate, p-toluenesulfonate, xylenesulfonate, tartarate, and the like. A preferred salt is the hydrochloride salt.

The pharmaceutically acceptable acid addition salts are typically formed by reacting a compound of formula I with an equimolar or excess amount of acid. The reactants are generally combined in a mutual solvent such as diethyl ether or benzene. The salt normally precipitates out of solution within about one hour to 10 days and can be isolated by filtration or the solvent can be stripped off by conventional means.

Bases commonly used for formation of salts include ammonium hydroxide and alkali and alkaline earth metal hydroxides, carbonates, as well as aliphatic and primary, secondary and tertiary amines, aliphatic diamines. Bases especially useful in the preparation of addition salts include ammonium hydroxide, potassium carbonate, methylamine, diethylamine, ethylene diamine and cyclohexylamine.

The pharmaceutically acceptable salts generally have enhanced solubility characteristics compared to the compound from which they are derived, and thus are often more amenable to formulation as liquids or emulsions.

Pharmaceutical formulations can be prepared by procedures known in the art. For example, the compounds can be formulated with common excipients, diluents, or carriers, and formed into tablets, capsules, suspensions, powders, and the like. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl pyrrolidone; moisturizing agents such as glycerol; disintegrating agents such as calcium carbonate and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonite; and lubricants such as talc, calcium and magnesium stearate, and solid polyethyl glycols.

The compounds can also be formulated as elixirs or solutions for convenient oral administration or as solutions appropriate for parenteral administration, for instance by intramuscular, subcutaneous or intravenous routes. Additionally, the compounds are well suited to formulation as sustained release dosage forms and the like. The formulations can be so constituted that they release the active ingredient only or preferably in a particular part of the intestinal tract, possibly over a period of time. The coatings, envelopes, and protective matrices may be made, for example, from polymeric substances or waxes.

The particular dosage of a compound of formula I required to inhibit primary endometrial hyperplasia or its symptoms, according to this invention, will depend upon the severity of the condition, the route of administration, and related factors that will be decided by the attending physician. Generally, accepted and effective daily doses will be from about 0.1 to about 1000 mg/day, and more typically from about 50 to about 200 mg/day. Such dosages will be administered to a subject in need thereof from once to about three times each day, or more often as needed, and for a time to effectively treat or prevent the disease or its symptoms.

It is usually preferred to administer a compound of formula I in the form of an acid addition salt, as is customary in the administration of pharmaceuticals bearing a basic group, such as the piperidino ring. For such purposes the following oral dosage forms are available.

### Formulations

In the formulations which follow, "Active ingredient" means a compound of formula I.

### Formulation 1: Gelatin Capsules

Hard gelatin capsules are prepared using the following:

| Ingredient | Quantity (mg/capsule) |
|---|---|
| Active ingredient | 0.1 - 1000 |
| Starch, NF | 0 - 650 |
| Starch flowable powder | 0 - 650 |
| Silicone fluid 350 centistokes | 0 - 15 |

The ingredients are blended, passed through a No. 45 mesh U.S. sieve, and filled into hard gelatin capsules.

Examples of specific capsule formulations of raloxifene that have been made include those shown below:

### Formulation 2: Raloxifene capsule

| Ingredient | Quantity (mg/capsule) |
|---|---|
| Raloxifene | 1 |
| Starch, NF | 112 |
| Starch flowable powder | 225.3 |
| Silicone fluid 350 centistokes | 1.7 |

### Formulation 3: Raloxifene capsule

| Ingredient | Quantity (mg/capsule) |
|---|---|
| Raloxifene | 5 |
| Starch, NF | 108 |
| Starch flowable powder | 225.3 |
| Silicone fluid 350 centistokes | 1.7 |

### Formulation 4: Raloxifene capsule

| Ingredient | Quantity (mg/capsule) |
|---|---|
| Raloxifene | 10 |
| Starch, NF | 103 |
| Starch flowable powder | 225.3 |
| Silicone fluid 350 centistokes | 1.7 |

### Formulation 5: Raloxifene capsule

| Ingredient | Quantity (mg/capsule) |
|---|---|
| Raloxifene | 50 |
| Starch, NF | 150 |
| Starch flowable powder | 397 |
| Silicone fluid 350 centistokes | 3.0 |

The specific formulations above may be changed in compliance with the reasonable variations provided.

A tablet formulation is prepared using the ingredients below:

### Formulation 6: Tablets

| Ingredient | Quantity (mg/tablet) |
|---|---|
| Active ingredient | 0.1 - 1000 |
| Cellulose, microcrystalline | 0 - 650 |
| Silicon dioxide, fumed | 0 - 650 |
| Stearate acid | 0 - 15 |

The components are blended and compressed to form tablets.

Alternatively, tablets each containing 0.1 - 1000 mg of Active ingredient are made up as follows:

### Formulation 7: Tablets

| Ingredient | Quantity (mg/tablet) |
|---|---|
| Active ingredient | 0.1 - 1000 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The Active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°-60° C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

Suspensions each containing 0.1 - 1000 mg of Active ingredient per 5 mL dose are made as follows:

### Formulation 8: Suspensions

| Ingredient | Quantity (mg/5 ml) |
|---|---|
| Active ingredient | 0.1 - 1000 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified water to | 5 mL |

The Active ingredient is passed through a No. 45 mesh U.S. sieve and mixed with the sodium carboxymethyl cellulose and syrup to form a smooth paste. The benzoic acid solution, flavor, and color are diluted with some of the water and added, with stirring. Sufficient water is then added to produce the required volume.

### ASSAYS

### ASSAY 1

### I. Primary Culture

Tissue from human or animal endometrial lesions are harvested and maintained in vitro as primary cultures. Surgical specimens are pushed through a sterile mesh or sieve or alternately teased or digested apart from surrounding tissue to produce a single cell suspension. Alternately cultures are propagated from tissue explants. Cells are maintained in growth media containing serum and antibiotics.

Rates of growth in the presence and absence of estrogen is determined. In vitro cultures are assessed for their proliferative response following treatment with estrogen, progestins, and compounds of Formula I. Levels of steroid hormone receptors are assessed to determine whether important cell characteristics are maintained in vitro. Tissue from 1-10 patients are utilized. In absence of a proliferative response, estrogen and anti-estrogen effects are assessed by monitoring transcriptional activation of estrogen regulated genes.

### II. Continuous Cultures

Continuous cultures of transformed endometrial cells reflective of endometrial hyperplasia and/or carcinoma are maintained in culture. Estrogen receptor containing cells (such as the Ishikawa line) are assessed for proliferation in response to estrogen and compounds of Formula I. Responsiveness is assessed by monitoring transcription of known estrogen/anti-estrogen regulated genes such as progesterone receptor, PS2 and others.

### ASSAY 2

I. Induced Animal Models - Uterine hyperplasia and carcinoma is induced by injections of estrogenic substances such as 17-β-estradiol or DES during neonatal development. The presence of hyperplasia or carcinoma in the adult animal is confirmed by biopsy or affected animals and/or sacrifice and biopsy of syngeneic animals identically treated. After evaluation of the lesions, affected animals are treated with estrogen, compounds of formula I, or progestins for 1-8 weeks. After treatment, lesions of surviving animals are examined for progression, regression or stasis.
II. Xenograft models - Implantation of endometrial lesions from humans or other animals or transformed cultured cells into immunodeficient rodents.

Tissue from endometrial lesions is implanted into the peritoneum or under the skin of sexually mature, castrated female immunocompromised rodents. Exogenous estrogen is supplied to induce growth of the explanted tissue. In some cases the harvested endometrial cells are cultured in vitro prior to implantation. Treatment consisting of estrogens, a compound of formula I, or vehicle will be supplied by gastric lavage on a daily basis for 3-16 weeks. Following treatment, implants are assessed for growth or regression. At the time of sacrifice, the uteri are harvested to assess the status of the intact endometrium.

### ASSAY 3

Five to fifty women are selected for the clinical study. The women suffer from primary endometrial hyperplasia, but otherwise are in good general health. The study has a placebo control group, *i.e*., the women are divided into two groups, one of which receives a compound of formula 1 as the active agent and the other receives a placebo. Women in the test group receive between 50-200 mg of the drug per day by the oral route. They continue this therapy for 3-12 months. Accurate records are kept as to the number and severity of the symptoms in both groups and at the end of the study these results are compared. The results are compared both between members of each group and also the results for each patient are compared to the symptoms reported by each patient before the study began.

Utility of the compounds of formula I is illustrated by the positive impact they have in at least one of the assays described above.

## Claims

1. Use of a compound having the formula 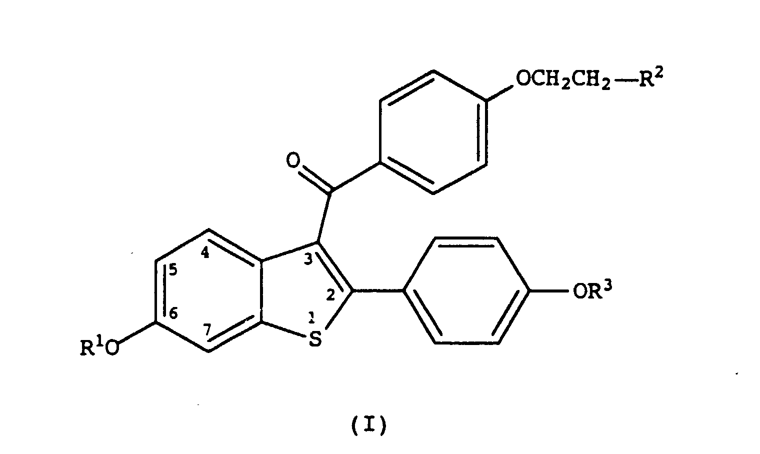 wherein R¹ and R³ are independently hydrogen, -CH₃, 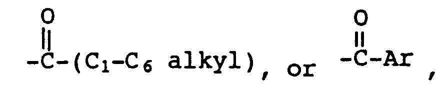 wherein Ar is optionally substituted phenyl;
R² is selected from the group consisting of pyrrolidine, hexamethyleneimino, and piperidino; or a pharmaceutically acceptable salt of solvate thereof, for the manufacture of a medicament for inhibiting primary endometrial hyperplasia.

2. The use according to Claim 1 wherein said compound is the hydrochloride sale thereof.

3. The use according to Claim 1 wherein said administration is prophylactic.

4. The use according to Claim 1 wherein said compound is 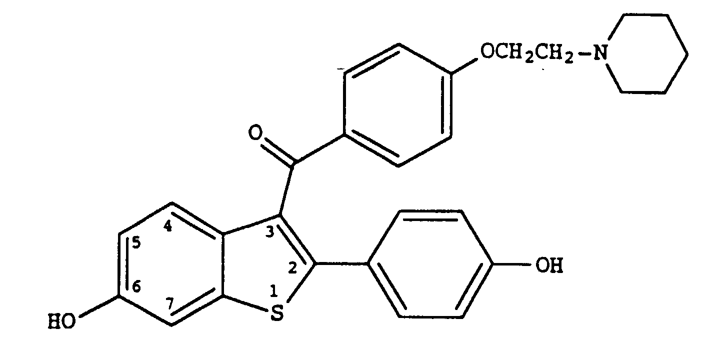 or its hydrochloride salt.

## Patentansprüche

1. Verwendung einer Verbindung der Formel 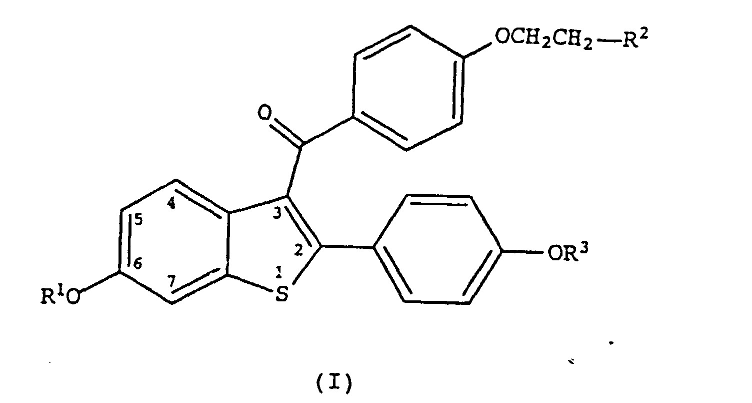 worin R¹ und R³ unabhängig für Wasserstoff, -CH₃, 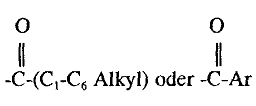 stehen, worin Ar für wahlweise substituiertes Phenyl steht,
R² aus der Gruppe ausgewählt ist, die aus Pyrrolidino, Hexamethylenimino und Piperidino besteht,
oder eines pharmazeutisch annehmbaren Salzes oder Solvats hiervon zur Herstellung eines Arzneimittels, das zur Hemmung der primären Endometriumhyperplasie brauchbar ist.

2. Verwendung nach Anspruch 1, worin die Verbindung das Hydrochloridsalz hiervon ist.

3. Verwendung nach Anspruch 1, worin die Verabreichung prophylaktisch ist.

4. Verwendung nach Anspruch 1, worin die Verbindung folgende ist 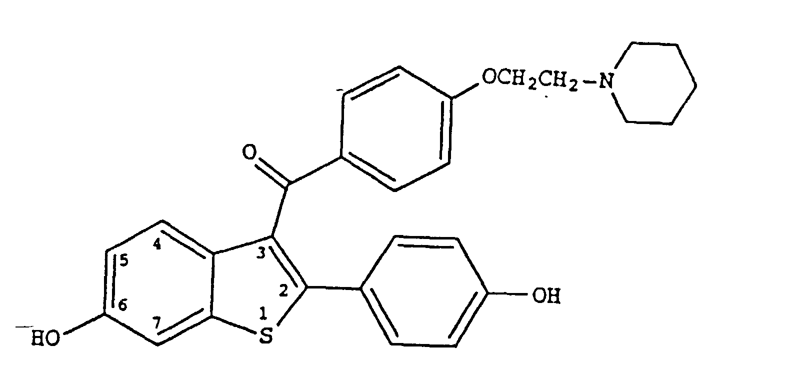 oder das Hydrochloridsalz hiervon.

## Revendications

1. Utilisation d'un composé répondant à la formule 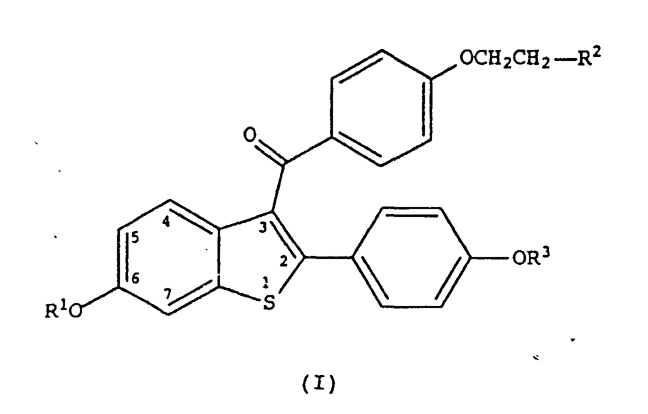 dans laquelle R¹ et R³ représentent, indépendamment l'un de l'autre, un atome d'hydrogène, un groupe -CH₃, un groupe 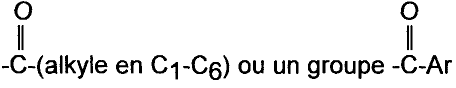 où Ar représente un groupe phényle le cas échéant substitué;
R² est choisi parmi le groupe constitué par un groupe pyrrolidino, un groupe hexaméthylène-imino et un groupe pipéridino; ou d'un de ses sels ou de ses solvates pharmaceutiquement acceptables, pour la formulation d'un médicament pour inhiber l'hyperplasie endométriale primaire.

2. Utilisation selon la revendication 1, dans laquelle ledit composé est son sel chlorhydrate.

3. Utilisation selon la revendication 1, dans laquelle ladite administration est de type prophylactique.

4. Utilisation selon la revendication 1, dans laquelle ledit composé est 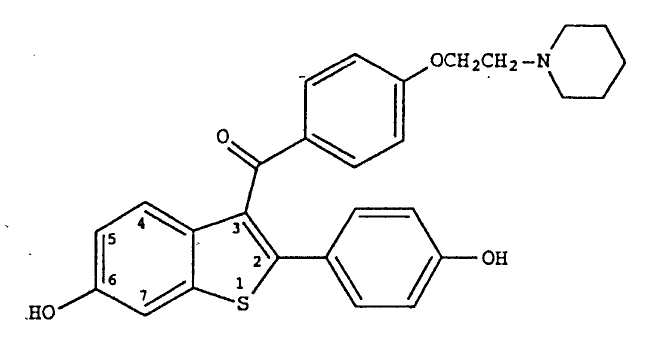 ou son sel chlorhydrate.
